# EUROPÄISCHE PATENTSCHRIFT

(11) **EP 2 903 570 B1**
(45) Veröffentlichungstag und Bekanntmachung des Hinweises auf die Patenterteilung: **12.06.2019**
(21) Anmeldenummer: 13771119.8
(22) Anmeldetag: 27.09.2013
(51) Int. Cl.: A61F 5/453, A61F 5/44, A61F 13/471

(54) **HYGIENEBEUTEL**
SANITARY BAG
SACHET HYGIÉNIQUE

(30) Priorität: 01.10.2012 DE 202012103745 U; 03.04.2013 DE 202013101413 U
(43) Veröffentlichungstag der Anmeldung: 12.08.2015
(73) Patentinhaber: McAirlaid's Vliesstoffe GmbH, 37339 Berlingerode (DE)
(72) Erfinder: MAKSIMOW, Alexander, 48565 Steinfurt (DE)
(74) Vertreter: Bungartz Christophersen Partnerschaft mbB Patentanwälte
(86) Internationale Anmeldenummer: PCT/EP2013/070241
(87) Internationale Veröffentlichungsnummer: WO 2014/053417

(56) Entgegenhaltungen:
- EP-A1- 0 787 472
- WO-A1-99/25281
- WO-A1-2004/071361
- JP-A- 2010 207 299
- US-A- 4 710 188
- US-A1- 2012 046 633
- US-B1- 6 338 729

## Beschreibung

Die vorliegende Erfindung betrifft einen Hygienebeutel zur Aufnahme zumindest eines Teils eines Körpergliedes, insbesondere des Penis.

Im Markt ist eine Vielzahl von Produkten zum Auffangen von Körperflüssigkeiten, wie z. B. Urin, bekannt. Zu erwähnen sind insbesondere Einlagen, Windeln für Säuglinge und Kleinkinder sowie Inkontinenzwindeln. Diesen Produkten ist gemeinsam, dass sie in der Regel als sogenannte Unisex-Produkte auf dem Markt sind, die Grundform ist an den Körperbau des Menschen angepasst. Es gibt darüber hinaus eine Vielzahl von speziell für Frauen entwickelte Produkte, wohingegen für Männer entwickelte Hilfsmittel nahezu unbekannt sind oder derart ausgestaltet sind, dass sie keinen Tragekomfort bieten.

Aus der DE 698 09 666 ist beispielsweise ein Urinauffangkissen für Männer bekannt, welches von einer mit einer Öffnung versehenen Beutel gebildet wird und aus mindestens einem Schichtstoffbogen besteht, welcher eine flüssigkeitsdurchlässige Innenlage, eine flüssigkeitsundurchlässige Außenlage und einen zwischen beiden Lagen vorgesehenen Absorptionskern aufweist. Dieser Beutel kann an einen Penis angebracht werden, indem der Penis in die Öffnung eingeführt wird, wobei flache oder linear elastische Elemente paarweise entlang einer Öffnungskante angeordnet sind, so dass die elastischen Elemente in einem knickverformten Zustand den Penis mittels elastischer Rückstellkräfte einklemmen.

In der DE 10 2008 020 606 B1 wird ein Hygieneartikel der eingangs genannten Art offenbart, welcher einen hütchenförmigen Grundkörper zum Aufsetzen auf das Körperteil aufweist, wobei der Grundkörper eine Öffnung aufweist, die größer ist als das Körperteil, und dieser Grundkörper eine steil ansteigende Flanke aufweist, die nach einem Scheitel in eine flach abfallende Kante übergeht, und der Grundkörper einen Überschlag aufweist, wobei die Öffnung verkleinerbar ist durch das Übereinanderschlagen des Überschlags über den über den Körperteil befindlichen Bereich des Grundkörpers. Üblicherweise ist der Grundkörper kegelförmig. Weitere Hygienebeutel sind zum Beispiel aus JP 2010 207299 A, US 4 710 188 A, US 2012/046633 A1, WO 2004/071361 A1 und EP 0 787 472 A1. US 6 338 729 B1 bildet den nächstliegenden Stand der Technik.

Den aus dem Stand der Technik bekannten Produkten ist gemeinsam, dass sie eine Form aufweisen, die ein industrielles Herstellungsverfahren aufwändig und somit teuer macht.

Der vorliegenden Erfindung lag somit die **Aufgabe** zugrunde, einen Hygienebeutel der eingangs genannten Art zur Verfügung zu stellen, der aus einfachen geometrischen Formen besteht und eine einfache und kostengünstige Herstellung auch mit Hochgeschwindigkeitsmaschinen ermöglicht.

Gegenstand der vorliegenden Erfindung ist somit ein Hygienebeutel zur Aufnahme zumindest eines Teils eines Körpergliedes, insbesondere des Penis, welcher Hygienebeutel zumindest aus einem Zuschnitt mit zwei Teilabschnitten A und B besteht, die jeweils die Form eines Vierecks haben und die entlang einer Faltlinie gefaltet sind, wobei die beiden Teilabschnitte von der Faltlinie bis zu ihrem der Faltlinie abgewandten Rand die gleiche Länge haben, dadurch gekennzeichnet, dass auf der Innenseite des Zuschnitts eine Saugeinlage angeordnet ist.

Der erfindungsgemäße Hygienebeutel besteht aus einem Zuschnitt, welcher entlang einer Faltlinie gefaltet ist, wobei diese gefaltete Form die äußere Form des Hygienebeutels bildet. Der Zuschnitt wird üblicherweise aus Bahnenmaterial erhalten. Um eine Herstellung in Hochgeschwindigkeitsmaschinen zu ermöglichen und auch den Verschnitt, also die nicht verwertbaren Reste des Materials, aus welchem der Zuschnitt herausgeschnitten wird, zu minimieren, weist der Zuschnitt eine möglichst einfache geometrische Form auf. Vorzugsweise haben die Teilabschnitte jeweils die Form eines Vierecks.

In einer bevorzugten Ausführungsform weist der Zuschnitt die Form eines Quadrats oder eines Rechteckes auf. Diese Form hat den Vorteil, da ein geringerer Verschnitt anfällt als bei anderen Formen.

Der Zuschnitt wird entlang der Faltlinie gefaltet, so dass die Teilabschnitte übereinanderliegen. Die der Faltlinie gegenüberliegenden Kanten des Zuschnitts bilden die Öffnung zur Aufnahme des Körperglieds. Erfindungsgemäß haben die Teilabschnitte von der Faltlinie bis zu ihrem der Faltlinie abgewandten Rand die gleiche Seitenlänge.

Liegt der Zuschnitt in Form eines Quadrates oder Rechteckes vor, also mit vier rechten Winkeln, so kann die Faltlinie sich in einem rechten Winkel zur Öffnung finden.

Die an die Faltlinie stoßenden und übereinanderliegenden Kanten der Teilabschnitte werden miteinander verbunden. Diese Verbindung kann in Abhängigkeit von dem Material, aus welchem der Zuschnitt besteht, unterschiedlich gestaltet sein. Beispielsweise können die Kanten durch Verkleben oder Verschweißen, in einigen Fällen auch durch Vernähen, miteinander verbunden werden. Diese Verbindung der Kanten ist wasserdicht, so dass ein Austreten der Körperflüssigkeit über diese Verbindung der Kanten weitgehend, möglichst vollständig, vermieden wird.

Erfindungsgemäß ist der Verbindungsrand von der Öffnungskante der Öffnung beabstandet, d. h. die Verbindung endet noch vor der Öffnungskante. Dadurch bleibt ein Abschnitt von der Schweißnaht bis zur Öffnung unverschlossen, wodurch die Öffnung erweitert wird. Durch diese Erweiterung wird das Einführen des Penis in die Öffnung erleichtert, also die Handhabung des erfindungsgemäßen Hygienebeutels.

Das Material, aus welchem der Zuschnitt besteht, sollte für Flüssigkeiten undurchlässig sein, es sollte vermieden werden, dass Urin oder andere Körperflüssigkeit durch den erfindungsgemäßen Hygienebeutel in Richtung Kleidung durchdringt und diese verschmutzt. Für einen optimalen Tragekomfort ist es bevorzugt, wenn der Zuschnitt aus einem atmungsaktiven Material besteht, d. h. aus einem Material, für Wasserdampf durchlässig ist, aber nicht für Flüssigkeiten. Das Material für den Zuschnitt kann beispielsweise eine atmungsaktive Folie sein, wie eine gelochte zwei- und/oder dreidimensionale Folie, ein atmungsaktives SMS, Nonwoven (Vlies) aus natürlichen oder synthetischen Fasern, und/oder ein Laminat aus unterschiedlichen Materialien, wie aus Nonwoven und atmungsaktiver Folie (BTBS-Filme = breathable film textile backsheet), wie sie auch für die Herstellung von Außenseiten von üblichen Inkontinenzprodukten bekannt sind.

Auf der Innenseite des Zuschnitts ist erfindungsgemäß eine Saugeinlage angeordnet. Die Saugeinlage erstreckt sich zumindest über einen Teil eines der Teilabschnitte, vorzugsweise erstreckt sich die Saugeinlage über beide Teilabschnitte. Vorzugsweise ist die Saugeinlage jedoch in Bezug auf die Ränder nach innen versetzt. Es können eine einzige oder mehrere Saugeinlagen auf dem Zuschnitt angeordnet werden. In einer bevorzugten Ausgestaltung erstreckt sich eine einzige Saugeinlage über beide Teilabschnitte A und b, wobei ihr Rand in Bezug auf die Ränder nach innen versetzt ist. Der Abstand zwischen der Saugeinlage und dem Rand sollte in Bezug auf die Ränder, die die aufeinanderliegende Verschlusskante bilden, so groß sein, dass sich die aufeinanderliegenden Ränder / Kanten miteinander verbinden lassen ohne die Saugeinlage einzufassen. Der Abstand der Saugeinlage bis zur Öffnungskante kann etwas größer sein und dieser Abstand bildet die Auflagefläche auf dem Körperglied und sollte so gewählt sein, dass ein guter Tragekomfort gewährleistet ist.

Der erfindungsgemäße Hygienebeutel ist mehrlagige aufgebaut und weist neben dem ersten Zuschnitt, der üblicherweise nach außen gerichtet ist, und der Saugeinlage auf der Innenseite, also in Richtung Körperglied, einen weiteren über der Saugeinlage angeordneten Zuschnitt auf. Dieser Zuschnitt ist aus einem für Flüssigkeiten durchlässigen Material. Die Körperflüssigkeit dringt durch diesen weiteren Zuschnitt in Richtung Saugeinlage. Aufgabe dieser weiteren Lage ist es, die Körperflüssigkeit schnell von dem Körperglied in Richtung Saugeinlage abzutransportieren, wo die Flüssigkeit aufgenommen und gespeichert wird. Der erste Zuschnitt und der weitere Zuschnitt weisen vorzugsweise die gleiche Größe auf. Die Saugeinlage wird von den beiden Zuschnitten umhüllt. Die beiden Zuschnitte können über eine geeignete Verbindung miteinander verbunden werden, beispielsweise durch Verschweißen mit Ultraschall. Es ist nicht erforderlich, dass die Zuschnitte wasserdicht miteinander verbunden werden. Die Zuschnitte sollten größer sein als die Saugeinlage, so dass beim Verbinden der Kanten der Zuschnitte die Saugeinlage nicht mit eingefasst wird.

Das Material aus welchem der weitere Zuschnitt besteht ist vorzugsweise ein für Flüssigkeiten durchlässiges Material, beispielsweise ein Vlies, vorzugsweise ein Polypropylen-/Polyethylen-Vlies, das dehnbar ist. Die Dehnbarkeit hat den Vorteil, dass es, wenn der Saugkörper Flüssigkeit aufnimmt und dessen Volumen zunimmt, auch nicht die Oberfläche dieses weiteren Zuschnitts verändert und dem Saugkörper Platz gibt, ohne dass dieser weitere Zuschnitt oder der nach außen gerichtete Zuschnitt zerreißt.

Aus diesem Grunde hat es sich auch als vorteilhaft erwiesen, wenn der Saugkern etwas kleiner ist als das Volumen, das der von dem äußeren Zuschnitt und dem weiteren Zuschnitt gebildete Volumen aufweist.

Diese Saugeinlage dient dazu, die Körperflüssigkeit aufzunehmen und dauerhaft zu absorbieren. Eine Rücknässung in Richtung Körperglied sollte vorzugsweise weitgehend vermieden werden. Diese Saugeinlage wird daher vorzugsweise von einem Material gebildet, welches dazu in der Lage ist, Körperflüssigkeiten, wie Urin, nicht nur zu absorbieren, sondern auch zu speichern. Gut geeignet sind Materialien aus gerichteten und/oder ungerichteten Fasern, insbesondere Vlies aus Zellstoff und Zellstoff-verwandten Materialien sowie synthetische saugfähige Materialien. Die Saugeinlage ist vorzugsweise ein Vlies aus Zellstofffasern, wie ein Airlaid.

Die Absorptionsfähigkeit des Saugkerns kann erhöht werden, wenn dieser Teilchen aus superabsorbierenden Polymeren enthält. Diese können beispielsweise direkt bei der Herstellung des Saugkerns eingearbeitet werden.

In einer möglichen Ausführungsform ist die Saugeinlage aus einer Faserstoffbahn aus Zellstofffasern oder einer im Luftstrom aufgeschichteten Schüttung von Zellstofffasern (Fluff Pulp) hergestellt, die durch einen aus zwei Prägewalzen bestehenden Kalander geführt werden. Unter Erzeugung eines Prägemusters im Druckbereich werden die Fasern bindemittelfrei punkt- oder linienförmig kalandriert und so verbunden. Als besonders geeignet hat sich ein Saugkern erwiesen, der gemäß dem in dem europäischen Patent 1 032 342 B1 beschriebenen Verfahren hergestellt wird.

Ein weiteres geeignetes Material ist ein Polymermaterial mit einer Wabenstruktur, wie es beispielsweise in der europäischen Patentanmeldung EP 2 444 046 A1 beschrieben ist.

Die Saugeinlage kann von einem Saugkörper wie oben beschrieben gebildet werden, dieser Saugkörper kann von flüssigkeitsdurchlässigen Lagen nach Art einer Hülle umgeben sein, wobei der Saugkörper an einer oder beiden Lagen fixiert sein kann oder nur lose von diesen Lagen umgeben ist.

Um ein Verrutschen der Saugeinlage auf dem Zuschnitt des Hygienebeutels zu vermeiden, kann die Saugeinlage auf dem Zuschnitt fixiert sein. Das Fixieren kann punktförmig oder vollflächig mithilfe von Klebstoff oder anderen adhäsiven Materialien erfolgen. In der Regel ist das Fixieren des Zuschnitts nicht erforderlich, die Reibung zwischen Zuschnitt und Saugkörper ist ausreichend.

In einer weiteren Ausgestaltung der vorliegenden Erfindung weist der erfindungsgemäße Hygienebeutel Mittel auf, um die Öffnung zu verkleinern und in ihrer Größe an den Umfang des Körperglieds anzupassen. Als geeignete Mittel können ein Zugband, ein elastisches Gummiband oder ein Bindemittel eingesetzt werden, mit welchem ein Verbindungsrand zum anderen umgeschlagen werden kann.

In einer bevorzugten Ausführungsform wird der eine Verbindungsrand zum anderen Verbindungsrand umgeschlagen und in dieser Stellung fixiert. Zum Fixieren kann der umgeschlagene Verbindungsrand beispielsweise Klebstoff oder ein anderes adhäsives Mittel aufweisen, das an dem Punkt, an welchem er fixiert wird, haftet. Vorzugsweise sollte die Haftung lösbar sein. In einer weiteren Ausgestaltung ist ein Bindemittel Überstand an dem umzuschlagenden Verbindungsrand angebracht, welcher ein entsprechendes Haftmittel aufweist, welches den umgeschlagenen Verbindungsrand fixiert und die Größe der Öffnung in ihrer Einstellung hält. Beispiele für geeignete Bindemittel sind elastisches und nicht-elastisches Klettband, elastisches und nicht-elastisches Klebeband usw. Diese Ausführungsform, in welcher ein Verbindungsrand zum anderen Rand umgeschlagen wird, hat den Vorteil, dass die obere Kante, die an dem Körperglied aufliegt, glatt bleibt und nicht gekräuselt wird, wodurch ein höherer Tragekomfort erzielt wird.

Die vorliegende Erfindung wird anhand der beigefügten Figuren näher erläutert.

Es zeigen:
- Fig. 1: eine perspektivische Darstellung eines gebrauchsfertigen Hygienebeutels;
- Fig. 2: eine Draufsicht auf eine erste nicht erfindungsgemäße Ausführungsform eines Hygienebeutels in noch nicht-gefalteter Form;
- Fig. 3: eine zweite nicht erfindungsgemäße Ausführungsform des Hygienebeutels in nicht-gefalteter Form;
- Fig. 4: eine dritte nicht erfindungsgemäße Ausführungsform des Hygienebeutels in nicht-gefalteter Form;
- Fig. 5: der Hygienebeutel nach Fig. 4, gefaltet entlang der Faltlinie;
- Fig. 6: der Hygienebeutel nach Fig. 4 in geschlossener Form;
- Fig. 7: eine dritte Ausführungsform des Hygienebeutels in noch nicht gefalteter Form;
- Fig. 8: die Ausführungsform nach Figur 7 in gefalteter, gebrauchsfertiger Form;
- Fig. 9: eine weitere Ausgestaltung des Hygienebeutels und Verbindungsnaht;
- Fig. 10: einen Schnitt entlang der Linie S-S in den Fign. 2, 3, 4 und 7;
- Fig. 11: eine vierte Ausführungsform des Hygienebeutels in noch nicht gefalteter Form;
- Fig. 12: die Ausführungsform nach Figur 11 in gefalteter, gebrauchsfertiger Form.

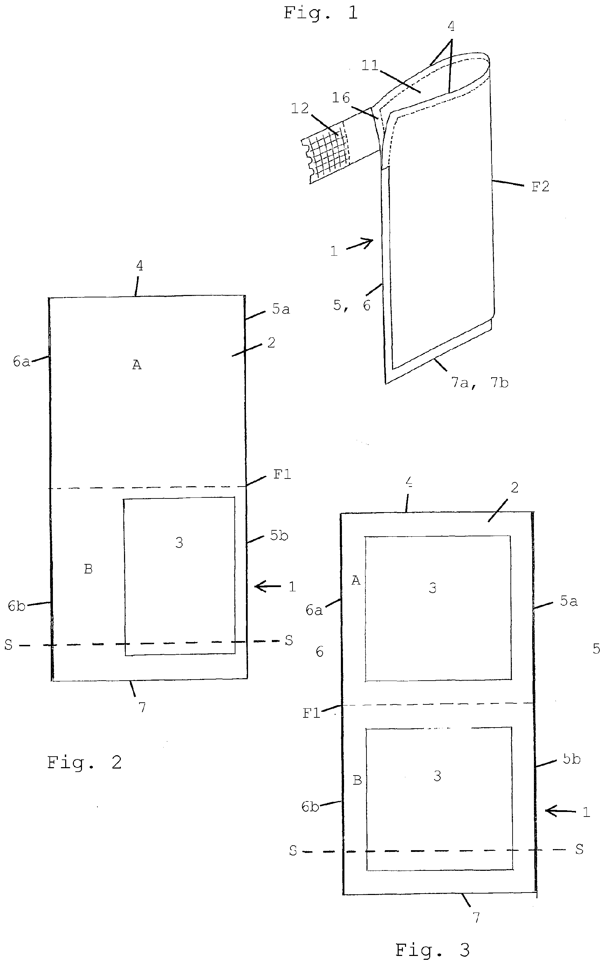

Fig. 2 zeigt eine mögliche Ausführungsform eines Hygienebeutels 1 in einem noch nicht gefalteten und noch nicht gebrauchsfertigen Zustand. Ein insgesamt viereckiger Zuschnitt 2 ist entlang einer späteren Faltlinie F1 in zwei zu der Faltlinie F1 symmetrische, rechteckige Teilabschnitte A und B unterteilt. In der hier dargestellten Ausführungsform ist nur auf dem Teilabschnitt B auf der späteren Innenseite eine Saugeinlage 3 angeordnet. Diese erstreckt sich von der Faltlinie F1 aus oder in geringem Abstand von der Faltlinie aus in Richtung zu der späteren Öffnungskante 4 des Hygienebeutels.

Der Zuschnitt 2 wird zunächst entlang der Faltlinie F1 umgeschlagen. Die dann jeweils aufeinanderliegenden Seitenkanten 5a und 5b bzw. und 6a und 6b werden dann in an sich bekannter Weise miteinander verbunden, beispielsweise durch Verkleben, Verschweißen oder Vernähen.

Durch das Falten und Verschließen bildet sich eine Tüte bzw. ein Beutel, der nur längs einer Kante bzw. eines Randes offen ist, d.h. eine Öffnung 11 hat. Die Öffnungskanten sind die Kanten 4 und 7. Die Faltlinie F1 bildet den Boden der Tüte und ist der Öffnung 11 abgewandt angeordnet.

In Figur 3 ist eine zweite mögliche Ausgestaltung des erfindungsgemäßen Hygienebeutels 1 dargestellt. Der Zuschnitt 2 selbst hat die gleiche Form wie bei Figur 2, jedoch befindet sich auf der späteren Innenseite beider Teilabschnitte A und B des Zuschnitts 2 jeweils ein flacher Saugkörper 3. Die Saugkörper 3 sind hier mittig in Bezug auf die Seitenkanten 5a, 6a und 5b, 6a angeordnet, sie können jedoch auch außermittig angeordnet sein.

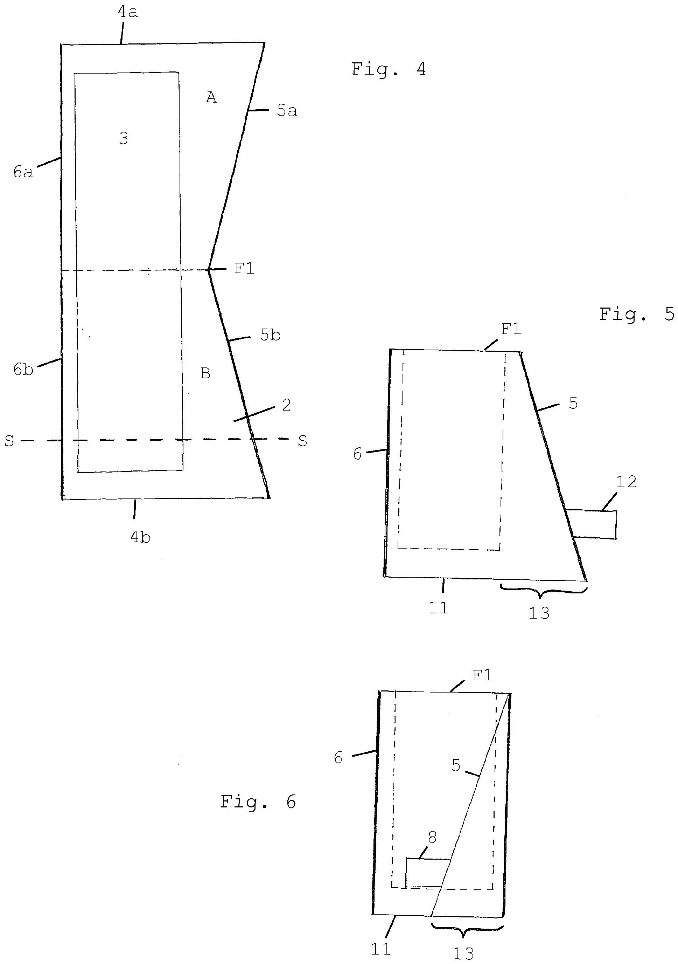

In Figur 4 ist eine dritte Ausgestaltung dargestellt, bei der der Zuschnitt 2 von zwei Teilabschnitten A und B in der Form jeweils eines Sehnentrapezes gebildet wird. Die kürzeren der beiden parallelen Trapezkanten stoßen an der Faltlinie F1 aneinander. Der Zuschnitt 2 kann, ebenso wie bei allen anderen Ausführungsformen, aus einem von einer Materialrolle abgerollten Bahnenmaterial bestehen.

In der in Figur 4 dargestellten dritten Ausführungsform erstreckt sich an der Innenseite des Zuschnitts 2 eine einzige Saugeinlage 3 quer über die Faltlinie F1, und über große Bereiche beider Teilabschnitte A und B. Der Zuschnitt 2 wird wiederum entlang der Faltkante F1 umgeschlagen. Die aufeinanderstoßenden Verbindungsränder 5a und 5b bzw. 6a und 6b werden in an sich bekannter Weise miteinander verbunden. Das Umschlagen des Zuschnitts 2 erfolgt so, dass sich die vorzugsweise als langes Rechteck gestaltete Saugeinlage 3 auf der Innenseite des gebildeten Hygienebeutels befindet. Die Öffnung 11 wird von den beiden Öffnungskanten 4a, 4b gebildet.

In Figur 5 ist der Hygienebeutel nach Figur 4 in seiner endgültigen, für den Gebrauch vorbereiteten Form dargestellt. Der Zuschnitt 2 ist entlang der Faltkante F1 umgeschlagen und die Verbindungsränder 5a, 5b, 6a, 6b liegen übereinander und sind in an sich bekannter Weise miteinander verbunden, wobei eine trapezgestaltige Tütenform mit der Öffnung 11 entsteht. Die flache, vorzugsweise als langes Rechteck gestaltete Saugeinlage 3 ist innenliegend angeordnet. Sie erstreckt sich über beide Teilabschnitte A und B und damit auch über den mit der Faltkante F1 zusammenfallenden Boden der Tüte. Jedoch ist die Saugeinlage 3 von solcher Länge, dass sie einen Abstand zu jeder der beiden Kanten 4a und 4b aufweist, die die Öffnung begrenzen.

Durch die zu der Öffnung 11 hin sich erweiternde Trapezform des Hygienebeutels wird ein Überschlag 13 in Gestalt eines Dreiecks gebildet. Dieser Überschlag 13 kann zum Gebrauch, wie in Figur 6 dargestellt, umgeschlagen werden, um so die Öffnung 11 auf die Größe des Körpergliedes einzustellen. Dazu wird der schräg zu der Öffnung 11 verlaufende Verbindungsrand 5 zu dem anderen Verbindungsrand 6 hin umgeschlagen, und dann in dieser Stellung mittels eines fixierenden Bindeelements 12 auf der Außenseite des Hygienebeutels befestigt.

Zur Anwendung wird der Hygienebeutel 1 in dem in Figur 5 dargestellten Zustand auf das Körperteil über die Öffnung 11 aufgebracht. Anschließend wird der dreieckige Überschlag 13 in Richtung zu dem anderen Verbindungsrand 6 gefaltet, so dass der vom Zuschnitt 2 gebildete Hohlraum das Körperteil, z. B. den Penis eines Mannes, fest umschließt. Daraufhin wird das Bindeelement 12 auf der Außenseite des Zuschnitts an geeigneter Stelle fixiert. Körperflüssigkeit, z. B. Urin, kann aus dem Körperteil austreten, gelangt dabei in den Hygienebeutel und wird von der Saugeinlage 3 absorbiert. Die Fixierung mittels des Bindeelements 12 ist vorzugsweise wieder lösbar, um den Hygienebeutel ohne Probleme wieder zu entfernen.

Der Hygienebeutel kann aber auch ohne besondere Bindeelemente oder Mittel zum Fixieren am Körperglied gehalten werden. Um die Öffnung 11 an den Umfang des Körpergliedes anzupassen und ggf. dem Anwender das Gefühl zu geben, dass der Hygienebeutel nicht verrutscht, können längs der die Öffnung 11 bildenden Ränder 4a und 4b oder in kurzem Abstand davon an der Außenseite oder Innenseite des Zuschnitts 2 elastische Materialien angebracht sein, die die Öffnung 11 mittels einer entsprechenden Vorspannung klein halten.

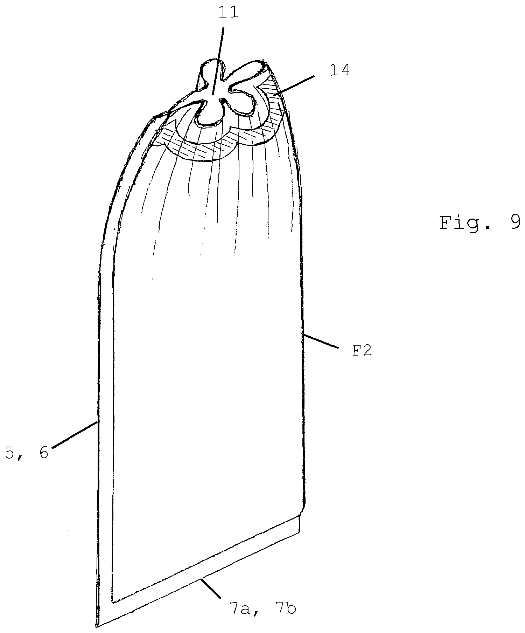

Gemäß Fig. 9 ist es auch möglich, entlang der Ränder 4a, 4b, also um die Öffnung 11 herum, ein elastisches, unter leichter Vorspannung in Umfangsrichtung stehendes Band durch diesen Randabschnitt des Zuschnitts zu legen, um so die Größe der Öffnung 11 variabel zu machen.

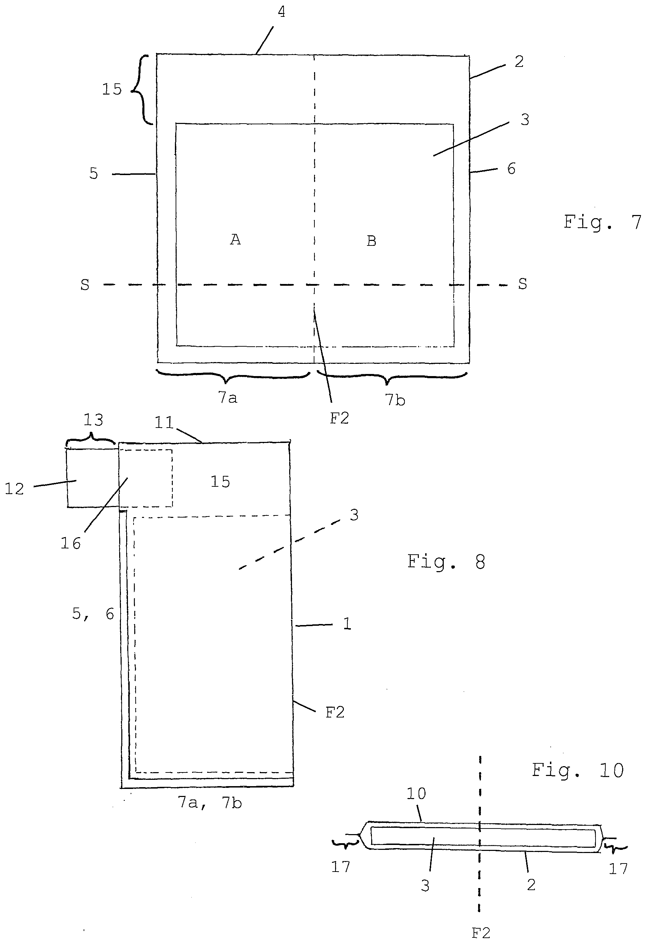

Bei der in Fig. 7 und Fig. 8 dargestellten Ausführungsform erstreckt sich die Saugeinlage 3 einteilig über beide Teilflächen A und B und über die quer zur Öffnung verlaufende Faltlinie F2 des Zuschnitts 2. Auch hier wird der Zuschnitt 2 entlang der Faltlinie F2 gefaltet, die mit der Kante 4 für die Öffnung einen rechten Winkel bildet. Die beiden seitlichen Kanten 5 und 6 sowie die beiden den Boden des Beutels bildenden Kanten 7a und 7b werden jeweils miteinander verbunden. In der hier dargestellten Ausführungsform erstreckt sich die Saugeinlage 3 nahezu über die ganze Höhe des Zuschnitts 2 und sie erstreckt sich insbesondere bis nahe an den durch die Kanten 7a, 7b gebildeten Boden. Zu den senkrechten Kanten 5 und 6 sowie zu den Bodenkanten 7a, 7b ist die Saugeinlage 3 allerdings soweit beabstandet, dass bei der Verbindung dieser Kanten 5, 6, 7a, 7b bzw. Randbereiche die Saugeinlage 3 nicht mit erfasst, also nicht mit eingebunden wird. Der Randbereich bis zur Öffnungskante 4 bildet eine Auflagefläche 15 des Hygienebeutels auf dem Körperglied. Über die Länge dieser Auflagefläche 15 wird auch die Größe der Öffnung 11 festgelegt.

Die Verbindungsränder 5, 6 bzw. 7a, 7b in der Ausführungsform nach den Fign. 1, 7 und 8 sowie die Verbindungsränder 5a, 5b, 6a, 6b in den Ausführungsformen nach den Fign. 2 bis 6 werden durch übliche, aus dem Stand der Technik bekannte Verfahren miteinander verbunden. Diese Verbindungsränder sollten vorzugsweise flüssigkeitsdicht sein, um ein Austreten von Körperflüssigkeit über diese Ränder zu verhindern. Eine solche Verbindung kann beispielsweise erhalten werden, indem die Kanten bzw. Ränder der Zuschnitte 2, 10 miteinander verscheißt werden. Das Verschweißen ist beispielsweise dann möglich, wenn die Zuschnitte aus thermoplastischem Material bestehen, die aufeinanderliegen Kanten werden durch Anwendung von Hitze angeschmolzen und verbinden sich, indem sie miteinander verschmelzen.

In der in Fig. 1 und Fig. 8 dargestellten, besonderen Ausgestaltung erstreckt sich die Verbindungskante entlang des Bodens und entlang der senkrechten Verbindungskanten 5, 6, die parallel zur Faltkante F2 verlaufen. Bei der in Fig. 1 und Fig. 8 dargestellten Ausführungsform erfolgt eine Verbindung der beiden senkrechten Ränder 5, 6, vom bodenseitigen Verbindungsrand 7a, 7b ausgehend, nicht auf ganzer Länge bis zur Öffnungskante 4. Vielmehr endet die Verbindung in einem Abstand noch vor der Öffnungskante 4, so dass ein Randabschnitt 16, der sich von der Schweißnaht bis zur Öffnung 11 erstreckt, unverschlossen bleibt. Dieser zur Seite hin unverschlossene Randabschnitt 16 wirkt wie eine Erweiterung der Öffnung 11, wodurch sich die Handhabung des Hygienebeutels vereinfacht.

Ebenfalls bei Fig. 1 und Fig. 8 kann in Höhe des Randabschnitts 16 entweder im Bereich der Kante 5 oder der Kante 6 ein Bindeelement 12 angebracht werden, dass zum Verschließen und Anpassen der Größe der Öffnung 11 auf der der Kante gegenüberliegenden Fläche 15 befestigt wird. Das Bindeelement 12 ist vorzugsweise wieder lösbar, beispielsweise ein Klettverschluss oder ein Klebeverschluss. Das Bindeelement 12 kann in seiner Längsrichtung elastisch ausgebildet sein, wodurch sich im Gebrauch die Größe der Öffnung 11 noch besser an das Körperglied anpasst.

Fig. 9 stellt eine Ausführungsform des Hygienebeutels dar, bei der die senkrechten Kanten 5, 6 bis an die Öffnung 11 heran verbunden, also geschlossen, sind. Das Anpassen der Größe der Öffnung an das Körperglied kann über ein an der Innenseite oder Außenseite des Hygienebeutels nahe oder direkt am Rand 4 angebrachtes elastisches Material erfolgen. In einer möglichen Ausgestaltung wird nahe des Randes 4 ein elastisch Zugband 14 eingezogen.

In der einfachsten Ausgestaltung weist der beutelförmige Hygienebeutel einen Zuschnitt 2 auf, in welchem ein Saugkörper 3 angeordnet ist. Der Saugkörper nimmt die abgegebene Körperflüssigkeit auf und speichert diese. Der Zuschnitt 2 soll verhindern, dass die Körperflüssigkeit nach außen, also in Richtung Bekleidung, austritt und die Bekleidung beschmutzt.

Ebenso können die erfindungsgemäßen Hygienebeutel in allen Ausführungsformen auch mehrlagig ausgestaltet sein. In einer möglichen Ausgestaltung ist ein weiterer Zuschnitt 10 innenseitig über dem Saugkörper 3 angeordnet. Dieser weitere Zuschnitt 10 bildet somit im Gebrauchszustand die Innenseite bzw. Innenwandung des Hygienebeutels. Hierzu ist in Fig. 10 ist ein Schnitt entlang der Linie S - S dargestellt und zeigt einen solchen dreilagigen Aufbau des Hygienebeutels. Der Saugkern 3 wird vom Zuschnitt 2, der die nach außen gerichtete Oberfläche des Hygienebeutels bildet und dem weiteren Zuschnitt 10, welcher die nach innen gerichtete Oberfläche des Hygienebeutels darstellt, gebildet. Die Kanten 5a, 5b und 6a, 6b sowie des Zuschnitts 2 sowie 5a, 5b und 6a, 6b des weiteren Zuschnitts sind in der Verbindung 17 miteinander verbunden und umschließen den Saugkörper 3. Das Verbinden der Kanten des Zuschnitts 2 und des weiteren Zuschnitts 10 kann in an sich bekannter Weise erfolgen. Es ist nicht erforderlich, dass diese Verbindung 17 wasserdicht ist. In einer bevorzugten Ausführungsform sind die Kanten locker aber nicht lösbar miteinander verbunden, so dass sich eine relativ weiche Verbindungskante 17 bildet. Im Bereich der Öffnungskante 4 liegt die Kante 17 auf dem Körperglied auf, insbesondere für diese Auflagefläche ist eine weiche und für die Haut angenehme Struktur von Vorteil. In dem in Fig. 10 dargestellten Schnitt ist der Saugkörper 3 von den Zuschnitten 2 und 10 umhüllt, ohne mit diesen verbunden zu sein. Es ist auch möglich, den Saugkörper 3 vollflächig oder teilflächig mit einem Zuschnitt 2, 10, oder gleichzeitig mit beiden Zuschnitten 2, 10 zu verbinden.

Eine weitere Möglichkeit den Saugkörper zu fixieren besteht darin, den Saugkörper 3 unbeweglich von den Zuschnitten 2 und 11 einzuschließen, die Verbindungskanten 5 und 6 also knapp und mit nur geringem Abstand zum Saugkörper zu fassen.

Wird der mehrlagige Aufbau nach Fig. 10 entlang der Faltlinien F1 oder F2 gefaltet, liegen die Verbindungen in den Rändern 5, 6 und 7a, 7b bzw. 5a, 5b und 6a, 6b übereinander und werden zur Fertigstellung des Hygienebeutels miteinander verbunden. Wie bereits beschrieben, ist diese Verbindung vorzugsweise wasserdicht.

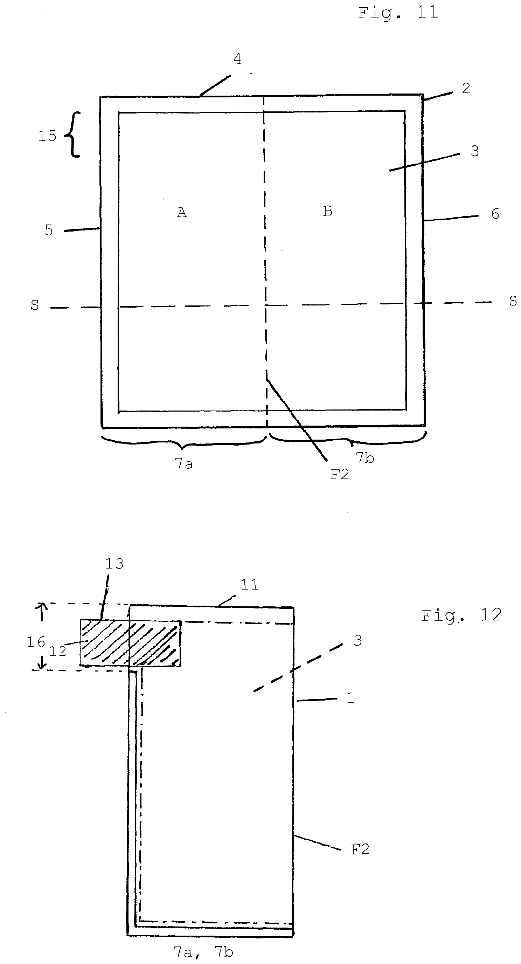

Die in Fig. 11 und Fig. 12 dargestellte Ausführungsform unterscheidet sich von der Ausfürhungsform aus Fig. 7 und Fig. 8 in der Anordnung der Saugeinlage 3. Auch in dieser Ausführungsform erstreckt sie sich einteilig über beide Teilflächen A und B und über die Faltlinie F2 des Zuschnitts 2. Die Saugeinlage reicht in Richtung Öffnungskante 4 über die Länge der Verbindungsnaht der Kanten 5, 6 hinaus. In der hier dargestellten Ausführungsform endet die Saugeinlage an der oberen Kante des Bindeelements 12.

Die Herstellung des erfindungsgemäßen Hygienebeutels erfolgt vorzugsweise aus Bahnenmaterial, das von einer Vorratsrolle abgewickelt der Maschine zugeführt wird. Das Material für den Zuschnitt 2, für die Saugeinlage 3 sowie für gegebenenfalls weitere vorhandene Lagen bzw. Zuschnitte 10, wird als auf Rollen aufgewickeltes Bahnenmaterial zugeführt. Die Saugeinlage 3 wird auf dem Bahnenmaterial des Zuschnitts 2 abgelegt, die gegebenenfalls vorhandene weitere Lage wird als weiterer Zuschnitt 10 gleichzeitig oder in einem nachfolgenden Schritt darüber angeordnet. In einem nächsten Verfahrensschritt werden die Saugeinlage 3 sowie das Bahnenmaterial für den Zuschnitt 2 und das Bahnenmaterial für den gegebenenfalls weiteren Zuschnitt 10 auf entsprechende Größe geschnitten. Zur Erleichterung des weiteren Herstellungsprozesses können die aufeinander liegenden Ränder miteinander verbunden werden, um so ein Verrutschen der aufeinander liegenden Zuschnitte zu verhindern. Das Verbinden kann beispielsweise mittels des oben bereits beschriebenen Ultraschallverschweißens erfolgen.

Anschließend wird der so produzierte Zuschnitt entlang der Faltlinie F1 bzw. F2 gefaltet, und die so übereinander gelangenden Kanten 5a, 5b und 6a, 6b bzw. 5, 6 und 7a, 7b werden unter Bildung einer wasserdichten Verbindung miteinander verbunden. Vorzgusweise wird noch zuvor, d.h. vor dem Herstellen der Verbindung der Kanten, das Bindeelement 12 oder das elastische Material 14 angebracht.

### Bezugszeichenliste

- 1: Hygienebeutel
- 2: Zuschnitt, außenliegende Lage
- 3: Saugeinlage
- 4: Öffnungskante
- 5: Verbindungsrand
- 5a, 5b: Verbindungsrand
- 6: Verbindungsrand
- 6a, 6b: Verbindungsrand
- 7: Rand, Öffnungskante
- 7a, 7b: Verbindungsrand
- 10: Zuschnitt, innenliegende Lage
- 11: Öffnung
- 12: Bindeelement
- 13: Überschlag
- 14: elastisches Material
- 15: Auflagefläche
- 16: nicht verschlossener Rand
- 17: Verbindung
- F1: Faltkante
- F2: Faltkante

## Patentansprüche

1. Hygienebeutel zur Aufnahme zumindest eines Teils eines Körpergliedes, insbesondere des Penis, bestehend zumindest aus einem Zuschnitt (2) mit zwei Teilabschnitten (A, B), die jeweils die Form eines Vierecks haben, wobei der Zuschnitt (2) aus einem für Flüssigkeiten undurchlässigen Material besteht, auf der Innenseite des Zuschnitts (2) eine Saugeinlage (3) angeordnet ist und auf der Innenseite über der Saugeinlage (3) ein weiterer Zuschnitt (10) aus einem für Flüssigkeiten durchlässigen Material angeordnet ist,
wobei die Zuschnitte (2, 10) entlang einer Faltlinie (F2) gefaltet sind, und
die nach Falten der Zuschnitte (2, 10) entlang der Faltlinie (F2) übereinanderliegenden Verbindungsränder (5, 6 und 7a, 7b) wasserdicht miteinander verbunden sind, wobei die beiden Teilabschnitte A und B von der Faltlinie (F2) bis zu ihrem der Faltlinie abgewandten Rand (5, 6) die gleiche Länge haben und, wobei der abgewandte Verbindungsrand (5, 6) von der Öffnungskante (4) einer Öffnung beabstandet ist und die Öffnung von der Öffnungskante (4) und von einem daran anstoßenden Abschnitt (16) gebildet wird, **gekennzeichnet dadurch, dass** der abgewandte Verbindungsrand (5, 6) parallel zur Faltlinie (F2) verläuft.

2. Hygienebeutel nach Anspruch 1, **dadurch gekennzeichnet, dass** die Zuschnitte (2 und 10) die gleiche Größe haben.

3. Hygienebeutel nach einem der Ansprüche 1 oder 2, **dadurch gekennzeichnet, dass** der Zuschnitt (2) aus einem atmungsaktiven Material ausgewählt ist aus gelochten Folien, atmungsaktiven SMS-Folien, Nonwoven aus natürlichen oder synthetischen Fasern, Laminaten aus Nonwoven und atmungsaktiven Folien und/oder BTBS-Folien.

4. Hygienebeutel nach einem der Ansprüche 1 bis 3, **dadurch gekennzeichnet, dass** das für Flüssigkeiten durchlässige Material ein elastisches Vlies ist.

5. Hygienebeutel nach einem der Ansprüche 1 bis 4, **dadurch gekennzeichnet, dass** sich eine einzige Saugeinlage (3) über beide Teilabschnitte A und B erstreckt, wobei ihr Rand in Bezug auf die Ränder (4, 5, 6) des Zuschnitts (2) nach innen versetzt ist.

6. Hygienebeutel nach einem der Ansprüche 1 bis 5, **dadurch gekennzeichnet, dass** die Zuschnitte (2 und 10) ausschließlich in Randbereichen miteinander verbunden sind.

7. Hygienebeutel nach einem der Ansprüche 1 bis 6, **dadurch gekennzeichnet, dass** die Saugeinlage (3) aus Bahnenmaterial aus gerichteten und/oder ungerichteten Fasern besteht, insbesondere aus Vlies aus Polymeren und/oder aus Zellstoff und Zellstoff-verwandten Materialien.

8. Hygienebeutel nach Anspruch 7, **dadurch gekennzeichnet, dass** die Saugeinlage (3) aus einer Faserstoffbahn aus Zellstofffasern oder einer im Luftstrom aufgeschichteten Schüttung von Zellstofffasern, die durch einen aus zwei Prägewalzen bestehenden Kalander geführt werden, hergestellt ist.

9. Hygienebeutel nach einem der vorangehenden Ansprüche, **dadurch gekennzeichnet, dass** am oberen Abschnitt des Verbindungsrandes (5, 6) ein Bindeelement (12) angeordnet ist.

10. Hygienebeutel nach einem der Ansprüche 1 bis 9, **dadurch gekennzeichnet, dass** der eine Verschlussrand (6) zu dem anderen Verschlussrand (5) umgeschlagen und in dieser Stellung fixiert wird.

11. Verwendung des Hygienebeutels nach einem der Ansprüche 1 bis 10 als Inkontinenzprodukt.

## Claims

1. Sanitary bag for receiving at least one part of the body, in particular the penis, which sanitary bag is composed at least of a blank (2) with two portions (A, B) which each have the shape of a quadrilateral, wherein the blank (2) is made of a material impermeable to liquids, an absorbent insert (3) is arranged on the inside of the blank (2) and a further blank (10) made of a material permeable to liquids is arranged on the inside over the absorbent insert (3), wherein the blanks (2, 10) are folded along a fold line (F2), and the connecting edges (5, 6 and 7a, 7b) superposed after folding the blanks (2, 10) along the fold line (F2) are connected to each other in a watertight manner, wherein the two portions A and B have the same length from the fold line (F2) to their edge (5, 6) facing away from the fold line, wherein the averted connecting edge (5, 6) is spaced apart from the opening edge (4) of an opening and the opening is formed by the opening edge (4) and by a portion (16) adjoining the latter, **characterized in that** the averted connecting edge (5, 6) runs parallel to the fold line (F2).

2. Sanitary bag according to Claim 1, **characterized in that** the blanks (2 and 10) have the same size.

3. Sanitary bag according to either of Claims 1 and 2, **characterized in that** the blank (2) which is made of a breathable material is chosen from perforated films, breathable SMS films, nonwovens of natural or synthetic fibers, laminates of nonwovens and breathable films and/or BTBS films.

4. Sanitary bag according to one of Claims 1 to 3, **characterized in that** the material permeable to liquids is an elastic nonwoven fabric.

5. Sanitary bag according to one of Claims 1 to 4, **characterized in that** a single absorbent insert (3) extends over both portions A and B, and its edge is offset inward with respect to the edges (4, 5, 6) of the blank (2).

6. Sanitary bag according to one of Claims 1 to 5, **characterized in that** the blanks (2 and 10) are connected to each other exclusively in edge areas.

7. Sanitary bag according to one of claims 1 to 6, **characterized in that** the absorbent insert (3) is made of web material from oriented and/or unoriented fibers, in particular of nonwovens from polymers and/or pulp and pulp-related materials.

8. Sanitary bag according to Claim 7, **characterized in that** the absorbent insert (3) is produced from a fibrous web of cellulose fibers or an airlaid bed of cellulose fibers which are guided through a calender composed of two embossing rollers.

9. Sanitary bag according to one of the preceding claims, **characterized in that** a binding element (12) is arranged on the upper portion of the connecting edge (5, 6).

10. Sanitary bag according to one of Claims 1 to 9, **characterized in that** the one closure edge (6) is turned back to the other closure edge (5) and is fixed in this position.

11. Use of Sanitary bag according to one of Claims 1 to 10 as an incontinence product.

## Revendications

1. Sachet hygiénique destiné à contenir au moins une partie d'un organe corporel, en particulier du pénis, se composant au moins d'une pièce (2) avec deux zones partielles (A, B), qui ont chacune la forme d'un carré, dans lequel la pièce (2) se compose d'un matériau imperméable aux liquides, une couche aspirante (3) est disposée sur le côté intérieur de la pièce (2) et une autre pièce (10) en un matériau perméable aux liquides est disposée sur le côté intérieur de la pièce (2), dans lequel les pièces (2, 10) sont pliées le long d'une ligne de pliage (F2), et les bords de jonction (5, 6 et 7a, 7b) superposés après le pliage des pièces (2, 10) le long de la ligne de pliage (F2) sont joints l'un à l'autre de façon étanche à l'eau, dans lequel les deux zones partielles (A, B) ont la même longueur de la ligne de pliage (F2) à leur bord (5, 6) éloigné de la ligne de pliage, et dans lequel le bord de jonction éloigné (5, 6) est espacé de l'arête d'ouverture (4) d'une ouverture et l'ouverture est formée par l'arête d'ouverture (4) et par une zone (16) jointive à celle-ci, **caractérisé en ce que** le bord de jonction éloigné (5, 6) est parallèle à la ligne de pliage (F2).

2. Sachet hygiénique selon la revendication 1, **caractérisé en ce que** les pièces (2 et 10) ont la même grandeur.

3. Sachet hygiénique selon une des revendications 1 ou 2, **caractérisé en ce que** la pièce (2) en un matériau respirant est sélectionnée parmi des feuilles perforées, des feuilles SMS respirantes, des non-tissés en fibres naturelles ou synthétiques, des stratifiés en non-tissés et feuilles respirantes et/ou des feuilles BTBS.

4. Sachet hygiénique selon l'une quelconque des revendications 1 à 3, **caractérisé en ce que** le matériau perméable aux liquides est une toile élastique.

5. Sachet hygiénique selon l'une quelconque des revendications 1 à 4, **caractérisé en ce qu'**une couche aspirante unique (3) s'étend sur les deux zones partielles (A, B), dans lequel leur bord est décalé vers l'intérieur par rapport aux bords (4, 5, 6) de la pièce (2) .

6. Sachet hygiénique selon l'une quelconque des revendications 1 à 5, **caractérisé en ce que** les pièces (2 et 10) sont jointes l'une à l'autre exclusivement dans les régions des bords.

7. Sachet hygiénique selon l'une quelconque des revendications 1 à 6, **caractérisé en ce que** la couche aspirante (3) se compose d'un matériau en bande en fibres orientées et/ou non orientées, en particulier en toile en polymères et/ou en cellulose et matériaux apparentés à la cellulose.

8. Sachet hygiénique selon la revendication 7, **caractérisé en ce que** la couche aspirante (3) est fabriquée en une bande de matière fibreuse en fibres de cellulose ou en un dépôt en vrac de fibres de cellulose formé dans un courant d'air, qui est mené à travers une calandre constituée de deux rouleaux de gaufrage.

9. Sachet hygiénique selon l'une quelconque des revendications précédentes, **caractérisé en ce qu'**un élément de liaison (12) est disposé à la partie supérieure du bord de jonction (5, 6).

10. Sachet hygiénique selon l'une quelconque des revendications 1 à 9, **caractérisé en ce qu'**un premier bord de fermeture (6) est replié vers l'autre bord de fermeture (5) et est fixé dans cette position.

11. Utilisation du sachet hygiénique selon l'une quelconque des revendications 1 à 10 comme produit pour incontinence.
